(19) Europäisches Patentamt
European Patent Office
Office européen des brevets

(11) Publication number: **0 166 505**
**A2**

(12) # EUROPEAN PATENT APPLICATION

(21) Application number: **85302252.3**

(22) Date of filing: **01.04.85**

(51) Int. Cl.⁴: **C 12 Q 1/42**, C 12 Q 1/32
// C12N9/16

(30) Priority: **25.06.84 US 624481**

(43) Date of publication of application: **02.01.86**
**Bulletin 86/1**

(84) Designated Contracting States: **BE CH DE FR GB IT LI SE**

(71) Applicant: **WARNER-LAMBERT COMPANY, 201 Tabor Road, Morris Plains New Jersey 07950 (US)**

(72) Inventor: **Lattime, Hollis C., 206 Stonegate Lane, Stanhope New Jersey 07874 (US)**

(74) Representative: **Jones, Michael Raymond et al, HASELTINE LAKE & CO. Hazlitt House 28 Southampton Buildings Chancery Lane, London WC2A 1AT (GB)**

(54) **Isolation and quantitation of alkaline phosphatase.**

(57) There is disclosed a process for isolating and quantitating an alkaline phosphatase comprising the steps of:

(1) separating the phosphatase from other enzymes in a biological sample;
(2) reacting the phosphatase from step (1) with a co-enzyme precursor to produce a co-enzyme;
(3) employing the co-enzyme produced in step (2) in a series of chemical reactions at least one of which uses a chromophore generation catalyst, to yield a chromophore, and
(4) measuring the absorbance of the chromophore which is generated in step (3).

EP 0 166 505 A2

0166505

HL 30103

# ISOLATION AND QUANTITATION OF ALKALINE PHOSPHATASE

Studies have shown that fast homoarginine-sensitive alkaline phosphatase (FHAP), a variant alkaline phosphatase, can be found in the biological samples taken from victims of various types of cancer. Typically, blood or serum from such patients contains abnormal levels of FHAP. Thus, the quantitative assay of FHAP can be a valuable tool in the diagnosis and treatment of cancer.

It has been discovered that FHAP can be isolated and quantitated via a combination of suitable separation and colorimetric assay techniques. In one embodiment, FHAP is separated from other enzymes or isoenzymes in a blood or serum sample by passing it through an ion exchange column which contains diethyl aminoethyl ion exchange resin in the form of crosslinked dextran. The resin in the column had been pretreated by equilibration using one or more buffer solutions containing a sodium azide preservative.

Using a pH 7.4 buffer solution as a carrier, a sample of human serum is passed through the pretreated column. The serum contains enzymes which can be characterized, based upon electrophoretic properties, as "fast" or "slow." The FHAP portion is the "fast" fraction while the other is "slower." The "slower" enzymes will wash through the column with the carrier. The "fast" portion, i.e., the portion which contains FHAP, is then eluted using additional buffer solution.

While column separation is a preferred method, the enzymes can be separated via electrophoresis, e.g., cellulose acetate or starch gel; immunological techniques using, e.g., polyclonal or monoclonal antibodies; and other separation methods.

The FHAP eluate is then contacted with a suitable substrate, e.g., nicotinamide adenine dinucleotide phosphate (NADP), under suitable conditions for the

commencement and continuance, if desired, of a cyclic oxidation-reduction reaction in which one of the reduction catalysts, e.g., diaphorase, generates a chromophore, e.g., reduced iodonitrotetrazolium (i.e., reduced INT or INTH). The absorbance of the chromophore can be measured by conventional colorimetric and/or spectrophotometric techniques.

It is an object of the invention to provide a process for quantitatively detecting an alkaline phosphatase via separation and colorimetric assay techniques.

It is another object of the invention to provide a composition useful in the colorimetric assaying of alkaline phosphatases.

The inventive process and composition have several advantages over prior art systems.

The treated ion exchange columns used to separate the alkaline phosphatase isoenzymes are stable and essentially ready to use. The buffers used in the pretreatment and separation are also stable reagents, which can be provided as concentrates, e.g., on the order of 10X, to reduce package bulk. In addition, the buffer solution used in the initial pretreatment step need not be measured (e.g., added to a mark on a resevoir), during pretreatment.

U.S. Patent 4,166,766 shows the separation of an alkaline phosphatase-containing serum using ion exchange columns which must be individually prepared daily. The columns of this invention require only resuspension of resin, adjustment of top filter height and draining of excess buffer prior to use.

The physical dimensions of the separation column of the invention are smaller than those used in U.S. Patent 4,166,766. Typically, about 2.0 mm height x about 0.8 mm diameter and about 1.0 ml bed volume are

needed in contrast to typical values of 1.65 mm height x 1.2 mm diameter and 2.0 ml bed volume.

The colorimetric assay is likewise more simple and efficient than prior art assays. The assay, in its simplest form, comprises adding a volume of sample to a volume of reagent, incubating for one or more short intervals (e.g., of about 30 minutes each), adding and mixing in one volume of 0.1 N HCl at timed intervals, and reading absorbance using a colorimeter or spectrophotometer.

The assay technique of the invention is more sensitive than other assays for alkaline phosphatase. According to the definition of enzyme units, 1 unit alkaline phosphatase converts 1 x $10^{-6}$ mole substrate (NADP) to product (NAD) per minute. Under the conditions of this procedure, 1 x $10^{-5}$ unit phosphatase produces sufficient final product (reduced INT) to give an absorbance (1 cm light path) of 0.016 to 0.020. As little as 2 x $10^{-6}$ units phosphatase gives a distinguishable difference in absorbance (0.0032 to 0.004).

It is believed that this high degree of sensitivity is due in part to the use of a highly effective "final chromophore generation catalyst", preferably an enzyme, e.g., diaphorase. The use of diaphorase or another enzyme-containing oxidation/reduction catalyst provides the assay with a lower blank reaction than would be possible with a light-sensitive chemical electron transfer reagent, e.g. phenazine ethosulphate, (PES) or phenazine methosulphate (PMS). The lower the blank absorbance, the easier it is to distinguish differences in absorbance due to sample activities.

A cyclic NAD/NADH reaction involving different oxidation/reduction catalysts is employed in the phosphatase determination system disclosed in European Patent Appln. 82300714.1, publication No. 0058539, published August 25, 1982.

While reagent blanks for the inventive system are

elevated, they are believed to be lower than those found using PES or PMS. In a 30 minute reaction period the reagent blank for the lactate dehydrogenase (LDH)/diaphorase combination of the invention is significantly lower than that of other oxidation-reduction catalyst combinations. One prior art combination using alcohol dehydrogenase (ADH) and PMS gives a blank of 0.416 while the instant LDH/diaphorase combination gives only 0.209 in the same reaction period.

Furthermore, the LDH/INT combination of the invention is more stable than prior art combinations. For example, a pH 9.6 reagent containing lactate and INT in aminomethylpropanol is stable for up to nine months at 20°C. The combined working reagent is stable for at least 7.5 hours at 2-8°C. LDH is generally less expensive than the ADH used in prior art systems.

The chromophore-generating catalyst of the invention, diaphorase, is a relatively expensive material. However, its cost is more than offset by its light stability and its compatibility with INT and LDH, which are less expensive than other conventional chromophore precursors and oxidation/reduction promoters.

One key feature of the invention is its storage stability and its ease of handling. Systems containing ADH cannot be used after 30 minutes. Systems containing non-enzyme chromophore generators are usually light sensitive, giving rise to background absorbances.

Finally, the instant invention can be used in a kit. Such a kit would provide FHAP-like controls for comparative purposes during the assay. Such controls are usually lyophilized materials designed to mimic FHAP's activity on the column and on electrophoresis.

Other aspects and advantages of the invention will become apparent from a consideration of the following description and claims.

Description of the Invention

The invention encompasses a novel composition and

a method of using it to assay for alkaline phosphatase content in a biological sample. It involves two broad processes: separation of the alkaline phosphatase and quantitation of same.

Separation

The biological samples from which FHAP or other alkaline phosphatase are to be separated are generally blood and blood components, e.g., serum. Other useful biological specimens on which the invention can be used include pleural fluid and ascites fluid.

The chromatographic separation carried out in accordance with the invention requires an anion exchange medium, optimally pretreated with buffer solution(s) to equilibrate it. pH values of about 7.0 to about 7.6, preferably about 7.2 to about 7.4, most preferably about 7.4, are used. The use of one suitable ion exchange resin is described in U.S. Patent 4,166,766.

One preferred separation scheme can be described as follows:

A control of FHAP-like material is produced by isolating a fraction of human placental alkaline phosphatase on a large scale FHAP column and adding it to bovine serum albumin (BSA). This control is to be used to mimic a biological sample for the separation and the assay.

Two buffer solutions were prepared.

Buffer I contains (in approximate amounts):

20 mM triethanolamine (TEA)
1.0 mM magnesium chloride
0.2 mM zinc chloride
0.05 g/100 ml sodium azide and
0.15 M sodium chloride.

Buffer I, at pH 7.4, is used to equilibrate the column.

Buffer II is the same as Buffer I except it contains about 0.4 M sodium chloride and about 0.025 g/100 ml sodium azide. 1.0 ml settled volume of resin

and approximately 1.0-1.5 ml equilibrating buffer were combined.

The treated column was used in the following separation scheme:

Separation method:

1. Apply 0.1 ml of sample or control (provided) to column.

2. Add 0.2 ml of Buffer I to rinse sample into resin.

3. Attach reservoir to top of column and add Buffer I to the mark.

4. Allow buffer to drain through column completely and discard all eluates to this point.

5. Place column over collection tube and add 4.0 ml of Buffer II.

6. Allow Buffer II to drain completely to yield FHAP eluate.

7. Mix each FHAP eluate thoroughly and assay for activity with the colorimetric assay.

The use of sodium azide preservative is a novel feature of this invention. When it is employed, it can be used in combination with suitable quantities of other preservatives. Typically sodium azide and or other preservatives will be used at total concentrations lying between about 0.01 and about 0.6 weight percent based on the weight of the total buffer solution. Typically Buffer I will contain about 0.04 to about 0.06 weight percent preservative, with Buffer II containing about 0.02 to about 0.03 weight percent preservative.

The pH value is an important feature of the separation step. Typically the anion exchange resin is first treated with Buffer I which has been adjusted to a pH of about 8.0 to about 8.4, preferably about 8.1 to about 8.3, most preferably about 8.15 to about 8.25 (product specifications call for 8.2 ± .05). The anion exchange resin is then treated one or more times with

Buffer I having a pH of about 7.2 to about 7.6, preferably pH of about 7.35 to about 7.45, until the pH of the equilibration buffer agrees to within + 0.01 pH units vs. Buffer I.

Buffer I concentrate, which is about pH 7.5 to about pH 7.6, preferably 7.55 ± .05, is diluted tenfold with distilled water to provide a working Buffer I with a pH of about 7.35 to about 7.45. Buffer II pH is also pH 7.35 to 7.45.

For the initial treatment of the column, the ratio of resin to buffer (i.e., weight/volume) will generally be from about 1 to about 20 mg/ml with about 5 to about 10 mg/ml preferred and about 8.4 mg/ml highly preferred.

During equilibration the ratio of resin to buffer will generally be about 1 to about 20 mg/ml, with about 7 to about 12 mg/ml preferred, and about 8.4 highly preferred. When equilibration is complete and the resin is filled into columns, the final resin/buffer ratio will be about 10 to about 60 mg/ml, preferably about 25 to about 50 mg/ml.

The use of substantial excesses of buffer is desirable for efficient equilibration. Other conventional recovery techniques can be used before, during, or after the isolation technique described above.

<u>Colorimetric Assay</u>

The reaction sequence employed in the assay of the invention involves reacting the isolated phosphatase eluate with a co-enzyme precursor to produce a co-enzyme and employing that co-enzyme in a series of cyclic chemical reactions during which a chromophore generation catalyst takes part in the production of a chromophore. The absorbance of the chromophore is then measured as a colorimetric assay of the amount of phosphatase taking part in or catalyzing the initial reaction.

While INT is the preferred chromophore precursor it can be completely or partially replaced with one or more other suitable reagents. Useful chromophore

generators include 3-(4',5'-dimethylthiazol-2-yl)-2,4-diphenyltetrazolium bromide (MTT), 2-(p-iodophenyl), 3-(p-nitrophenyl)-5-phenyltetrazolium chloride (INT), 2,2',5,5'-tetra-(p-nitrophenyl)-3,3'-(3,3'-dimethoxy-4,4'-diphenylene)diphenyltetrazolium chloride (TNBT), 2,2'-di(p-nitrophenyl)-5,5'-diphenyl-3,3'(3,3'-dimethoxy-4,4'-diphenylene)ditetrazolium chloride (NBT), 2,2'-p-diphenylene-3,3',5,5'-tetraphenylditetrazolium chloride (neotetrazolium chloride) (NT), 2,3-5-triphenyltetrazolium chloride (TT) and the like.

The chromophore generation catalyst can be any catalytic agent having the requisite light stability and compatibility with the other reagents in the system. The preferred chromophore generation catalyst is a diaphorase. Useful diaphorases include that obtained from Clostridium kluyveri or other suitable sources. Mixtures are operable.

Using one preferred group of reagents, a typical assay can be represented by the following scheme:

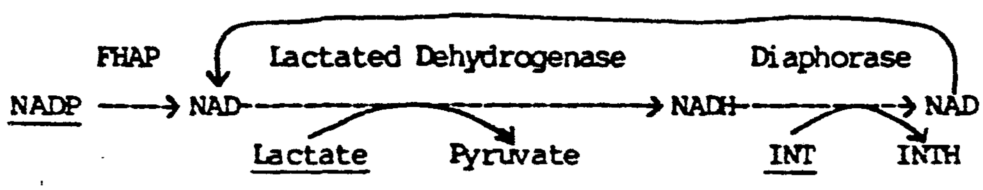


The absorbance of the INTH (reduced iodonitrotetrazolium or INT formazan) is a measure of the FHAP activity. The NAD is recycled so that for each molecule of NADP cleaved by FHAP, numerous molecules of INTH are formed. This cycling reaction can continue until one or more of the underlined constituents are depleted, until product inhibition hampers any of the enzymatic reactions or until the reaction is stopped, for example, by a change in pH.

Table I gives the concentrations of the chemical components employed in the reaction mixture of the instant assay. The "Substrate Reagent" column gives values for substances as they would be packaged for shipment or storage. Lyophilization and/or other

stabilizing techniques will have been performed thereon. The "Reconstituting Reagent" column is self-explanatory. The "Reaction Mixture" is the final formulation which is used for the assay technique.

## Table I

| | Substrate Reagent | Reconstituting Reagent | Reaction Mixture* |
|---|---|---|---|
| Tris buffer** | 10.0 mM | -- | 5.0 mM |
| Methyl paraben (Preservative) | 0.02% | 0.02% | 0.04% |
| HEDTA | 4.0 mM | -- | 2.0 mM |
| Zinc chloride | 1.8 mM | -- | 1.0 mM |
| Magnesium chloride | 2.0 mM | -- | 1.5 mM |
| Bovine Serum Albumin | 0.4% | -- | 0.20% |
| NADP | 0.1 mM | -- | 0.05 mM |
| Diaphorase*** | 330 U/L | -- | 165 U/L |
| LDH | 10000 U/L | -- | 5000 U/L |
| INT | -- | 1.5 mM | 0.75 mM |
| 2-A-2-M-1-P buffer**** | -- | 200 mM | 100 mM |
| L-Lactic acid | -- | 200 mM | 100 mM |
| pH | pH 7.8 (lyophilization pH) | pH 9.6 | pH 9.5 (reconstituted pH) |

The notations identified in Table I represent the following:

* The reaction mixture is made up of equal volumes of reconstituted substrate reagent and sample in triethanolamine buffer, 20 mM, pH 7.4 with 1.0 mM magnesium chloride, 0.2 mM zinc chloride, 400 mM sodium chloride and 0.025% sodium azide.

** Trishydroxymethylamino methane

*** From Clostridium kluyveri (e.g., from Worthington Diagnostic Systems, a division of Cooper Biomedical)

**** 2-Amino-2-Methyl-1-Propanol

A typical FHAP assay is performed as follows:

FHAP is separated by ion exchange chromatography. A 0.1 ml serum sample yields 4.0 ml FHAP eluate.

1. 1.0 ml of FHAP eluate or standard is preincubated 2 min. at 37°C.

2. On timed intervals, 1.0 ml of reconstituted substrate reagent is added per sample, mixed and incubated at 37°C.

3. After 30 min., and on the same timed intervals, 1.0 ml of 0.1 N HCl is added to stop the reaction.

4. The reaction mixture is mixed thoroughly and the absorbance read at about 480 to about 520 nm, preferably at about 500 nm.

5. The FHAP activity is calculated from the standard curve. The standard curve should be run at least once for a given lot of reagents.

Generally at least 4 tubes/run (Blank, normal control, elevated control, 1 sample) will be used. However up to 120 tubes can be run at once (30 min. x 4/minute).

This procedure is sufficiently sensitive to measure normal and elevated levels of FHAP activity. The cutoff for normal FHAP activity is approximately 2.1 U/L. The exact cutoff is to be determined. It appears that the cutoff for the colorimetric assay may be about 2.5. Because the sample is diluted one to forty in the FHAP separation, this means that the described colorimetric assay is capable of measuring 2.1/40 or 0.05 U/L FHAP activity. Normal levels of alkaline phosphatase activity are in the range of 50-100 U/L, therefore the above assay is significantly more sensitive than a typical alkaline phosphatase assay.

Conventional methods for reading and/or recording the depth of color of the INTH or other chromophore can be used. Typically, colorimeters and spectrophotometers such as Fisher Electrophotomate II or Gilford 300-N are employed.

At 500 nm, typical absorbances of FHAP are as given in Table II:

Table II

| U/L FHAP | A500 |
|---|---|
| 0 | 0.221 |
| 3.5 | 0.366<br>0.369 |
| 7.0 | 0.505<br>0.505 |
| 14.0 | 0.786<br>0.780 |
| 28.0 | 1.322<br>1.324 |

Other quantifying methods can be employed in combination with the method described above.

A typical FHAP separation scheme can be described as follows:

A. Column preparation

1. Resuspend resin by setting column upside down on stopper for a few minutes.

2. Turn column upright and remove stopper.

3. Set column in appropriate rack over a receptable suitable for collecting about 32 ml of waste buffer per column.

4. Remove bottom tip cap. Resin will settle in 20-60 seconds.

5. Using an appropriate plunger (may be provided, or use blunt end of Pasteur pipette or glass rod etc.), push top filter disc down to the level of the resin (2 cm $\pm$ 0.5 cm bed height). Do not compress resin, to achieve good flow rate.

6. Allow excess buffer to drain completely before sample application. Column can be drained up to within 30 minutes of sample application.

B. Reagent Preparation

1. Buffer I: Dilute 80 ml Buffer I concentrate to 800 ml with distilled or deionized water. Mix well. The reagent should be at about room temperature (15°-30°C) before use.

2. Normal Control: Reconstitute one vial with 1.0 ml of distilled or deionized water. Allow about 1 to about 10, preferably about 5 minutes of complete dissolution and mix well. The control is stable for at least four hours at 25°C or 48 hours at 2-8°C.

3. The Elevated Control is prepared in the same manner as the Normal Control.

II. FHAP Separation Procedure

0.1 ml each of a serum sample, normal control and elevated control were applied to columns and allowed to drain into the resin. 0.2 ml of buffer I was added to each and allowed to drain into the resin, rinsing the samples into the resin. The supplied reservoirs were each attached securely to the column tops and Buffer I was added to the mark (9 mm from the reservoir top). The columns were allowed to drain completely (40-60 minutes). Any fluid clinging to the columns was wiped off and the columns were set upright over appropriately test labeled tubes in a rack. 4.0 ml of Buffer II was added to each column and the columns were allowed to drain completely (5-10 min.) Each tube was mixed thoroughly and saved for the color assay (eluates stable 24 hours at 25°C or up to 3 days at 2-8°C).

III. FHAP Color Assay

Reagent Preparation

1. Substrate Reagent

Reconstitute each 5 test vial with 5 ml (10 test - 10 ml etc.) of 2-8°C reconstituting reagent, mix thoroughly and store on ice until use (reconstitute at least 15 minutes before use. The reagent is stable at least 7.5 hours at about 2° - 8°C).

2. FHAP Standards

Reconstitute one vial of FHAP standard with 5.0 ml of Buffer II. Allow 10 minutes to dissolve completely and mix well. Into approximately labeled test tubes pipette e.g., 4, 2, 3 and 0 ml of Buffer II. Into these same tubes, pipette respectively 0, 2, 1 and remainder of FHAP standard. Mix the two intermediate standards thoroughly. Store at 2-8°C and use in the same manner as sample eluates and control eluates.

Color Assay Procedure - with sample preincubation

1. Pipette 1.0 ml of each sample eluate, control eluate on FHAP standard into labeled test tubes.

2. On timed intervals, for example, every 15 seconds, place one sample tube in a 37° ± 1°C incubating bath or block until all tubes are incubating.

3. Starting two minutes after pre-incubation of the first tube, pipette 1.0 ml of substrate reagent into the first tube. Mix and incubate at 37°C. Using the same timed intervals used previously, continue until reagent has been added to all tubes.

4. 30 minutes following substrate addition, and on the same intervals, add 1.0 ml of 0.1N HCl and mix. Continue until HCl has been added to all tubes.

5. Mix each tube thoroughly and read absorbance at 500 nm.

Color Assay Procedure - without sample preincubation

1. Pipette 1.0 ml of each sample eluate, control eluate on FHAP standard into labeled test tubes.

2. On timed intervals, e.g., every 15 seconds, add 1.0 ml of substrate reagent to each tube, mix and incubate at 37° ± 1°C.

3. 30 minutes following substrate addition, and on the same intervals, add 1.0 ml of 0.1N HCl and mix. Continue until HCl has been added to all tubes.

4. Mix each tube thorough and read absorbance at 500 nm.

Calculation of FHAP Activity:

1. Graph absorbance vs. FHAP standard activities on rectilinear graph paper.

2. Read FHAP activities for samples and controls from standard curve using absorbance.

or

1. Using suitable calculator, obtain linear regression analysis of standard curve.

2. Calculate sample or control activities from absorbances.

Table III gives typical values for a variety of sera.

Table III

Normal Sera

| Sample | Color (U/L) |
|---|---|
| Pregnant woman, 3rd trimester | 1.74 |
| Smoker #1 | 0.97 |
| Diabetic | 2.09 |
| Control #1 | 1.52 |
| Smoker #2 | 1.41 |
| Smoker with arthritis | 1.34 |
| Control #2 | 0.90 |
| Control #3 | 0.97 |

Diseased (Abnormal) Sera

| Sample | Color (U/L) |
|---|---|
| Colon Cancer Metastatic to Liver | 3.72 |
| Breast Cancer | 3.80 |
| Colon Cancer #1 | 46.83 |
| Lung Cancer | 15.82 |
| Colon Cancer #2 | 33.14 |
| Rectal Cancer | 23.54 |

These values were derived via the above method using 6.24 units/liter FHAP (i.e., 78 units/liter alkaline phosphatase) diluted in Buffer II and a DuPont ACA instrument with alkaline phosphatase pack.

Reasonable variations, such as those which would occur to a skilled artisan, can be made herein without departing from the scope of the invention.

I claim:

1. A process for isolating and quantitating an alkaline phosphatase comprising the steps of:

(1) separating the phosphatase from other enzymes in a biological sample;

(2) reacting the phosphatase from step (1) with a co-enzyme precursor to produce a co-enzyme;

(3) employing the co-enzyme produced in step (2) in a series of chemical reactions at least one of which uses a chromophore generation catalyst, to yield a chromophore, and

(4) measuring the absorbance of the chromophore which is generated in step (3).

2. The process of Claim 1 wherein the phosphatase is fast homoarginine-sensitive alkaline phosphatase and the chromophore generation catalyst is a diaphorase.

3. The process of Claim 1 or 2 wherein step (1) is carried out via chromatography using an anion exchange medium.

4. The process of Claim 3 wherein the anion exchange medium is an ion exchange resin containing diethyl aminoethyl resin in a crosslinked dextran form.

5. The process of any one of Claims 1 to 4 wherein the chromophore is reduced iodonitrotetrazolium.

6. The process of any one of Claims 1 to 5 wherein step (3) is carried out using lactate dehydrogenase.

7. The process of any one of Claims 1 to 6 wherein step (2) is carried out using nicotinamide adenine dinucleotide phosphate.

8. The process of Claim 3 or Claim 4, 5, 6, or 7 when appendant to Claim 3, wherein the ion exchange medium has been treated with a preservative.

9. The process of Claim 8 wherein the preservative is a paraben.

10. A composition useful in the quantitation

of a co-enzyme which precusor reacts in the presence of alkaline phosphatase, at least one oxidation/reduction catalyst for the co-enzyme, and at least one chromophore-generating oxidation/reduction catalyst.

11. The composition of Claim 10 wherein the chromophore-generating oxidation/reduction catalyst is a diaphorase.

12. The composition of Claim 10 or 11 wherein the co-enzyme precursor is nicotinamide adenine dinucleotide phosphate, and the oxidation/reduction catalyst for the co-enzyme is lactate dehydrogenase.

13. The composition of Claim 10, 11 or 12 further comprising a buffer, a preservative, and a chromophore precursor.

14. The composition of claim 13 wherein the chromophore precursor comprises iodonitrotetrazolium.

15. A composition useful for the isolation of fast homoarginine-sensitive alkaline phosphatase from a biological sample comprising: an anion exchange medium which has been treated with a preservative.

16. The composition of Claim 15 wherein the preservative comprises sodium azide.

17. The composition of Claim 15 or 16 wherein the anion exchange medium is an ion exchange resin containing diethyl aminoethyl resin in a crosslinked dextran form.